# EUROPEAN PATENT APPLICATION

(11) **EP 2 679 951 A2**
(43) Date of publication of application: **01.01.2014**
(21) Application number: 13397522.7
(22) Date of filing: 19.06.2013
(51) Int. Cl.: G01B 11/245

(54) **System for imaging sawn timber**

(30) Priority: 27.06.2012 FI 20125733
(71) Applicant: Fin Scan Oy, 02201 Espoo (FI)
(72) Inventor: Karinen, Sakari, 02870 Espoo (FI)
(74) Representative: Tiilikainen, Jarkko Tapio

(57) **Abstract**

System for imaging pieces of sawn timber (3) travelling transversely past the system, in which each piece of sawn timber (3) comprises a top surface and a bottom surface, as well as sides connecting them, and which system is arranged to image the top surface and at least one side when the sawn piece (3) travels past the system's measuring area (7). According to the invention, the system comprises at least one matrix camera (5) for imaging a sawn piece (3), which is positioned in a first position relative to the path of the sawn pieces (3). In addition, the system comprises at least one mirror (6), which is positioned in a second position relative to the path of the sawn pieces (3) and to the said first position. According to the invention, the first and second positions are selected in such a way that each sawn piece (3) travelling past transversely can be imaged using the said at least one matrix camera (5) directly from a first direction and as a mirror image through the said at least one mirror (6) from at least one second direction.

## Description

The invention relates to a system, according to the preamble to Claim 1, for imaging sawn timber.

### Prior art

Publication WO 00/62011 A1 discloses an apparatus for determining the dimensions and external properties of timber using the so-called transverse-measurement method.

In the transverse-measurement method, the sawn timber travels past the apparatus transversely, i.e. the apparatus images the pieces of sawn timber essentially simultaneously over their entire length. Correspondingly, the so-called longitudinal method is also known, in which the sawn pieces pass the imaging apparatus in their longitudinal direction, in which case the apparatus images the sawn pieces at one time over their entire width. In the longitudinal method, the transportation of sawn timber through the apparatus takes longer however, as pieces of sawn timber are typically considerably longer than they are wide.

In the apparatus described in publication WO 00/62011 A1, the object being measured is examined in transverse measurement in a single measurement station using the same illumination arrangement, by means of colour-line cameras from three different directions, preferably using colour-line cameras. The apparatus indeed comprises at least three such cameras.

### Description of the invention

The invention is intended to create a new solution for the imaging of sawn timber.

The invention is based on the piece of sawn timber passing the measurement area being imaged using a matrix camera simultaneously both directly and with the aid of at least one mirror image formed through at least one mirror, in which case image material is assembled simultaneously from at least two, and in some embodiment even three faces of the piece of sawn timber.

More specifically, the method according to the invention is characterized by what is stated in the characterizing portion of Claim 1.

With the aid of the invention, a new solution is created for the imaging of sawn timber.

The invention also makes possible several different embodiments, which offer additional advantages.

For example, the invention has embodiments, in which the number of cameras can be reduced compared to known solutions. This is because with the aid of the embodiments the same measurement element can be used to image all three faces of a piece of sawn timber.

The invention also has embodiments, which facilitate the calibration of the cameras.

The invention also has embodiments, which permit a more compact measurement area than known solutions, making it also easier to implement the illumination of the measurement area. At the same time, the measurement conveyor can also be implemented in a shorter form.

### Description of the Figures

In the following, the invention is examined with the aid of examples and with reference to the accompanying drawings.
Figure 1 shows a schematic drawing of a top view of the arrangement according to one embodiment.
Figure 2 shows a schematic drawing of a side view of the arrangement according to one embodiment.
Figure 3 shows an example of the creation of an image in one embodiment.
Figure 4 shows a schematic drawing of a side view of the arrangement according to a second embodiment.

### Embodiments of the invention

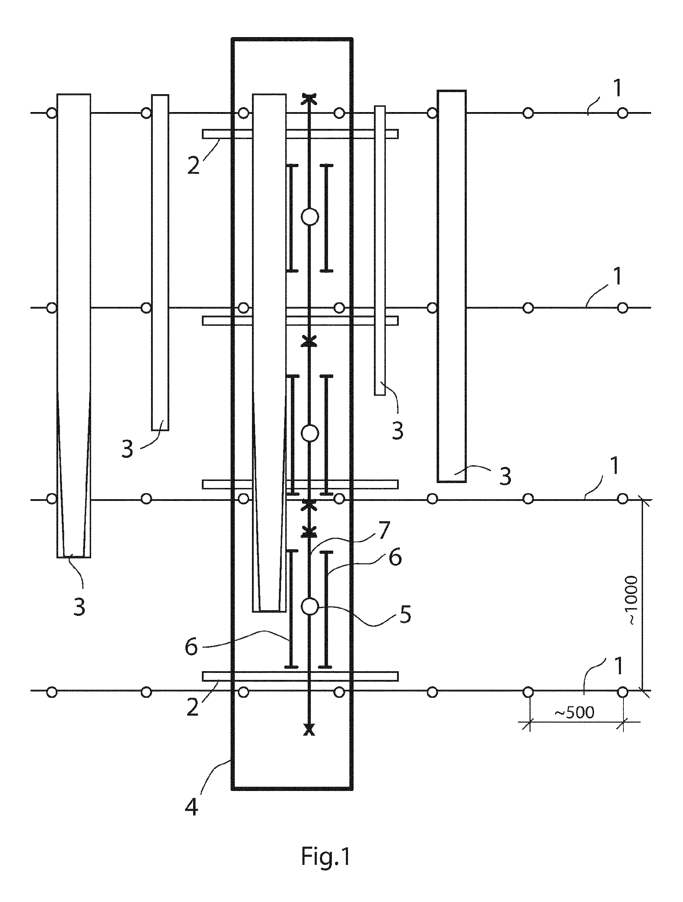

The system of Figure 1 comprises a main conveyor 1, which includes a chain and slats for transporting pieces of sawn timber 3 transversely to the measurement conveyor 2 and then away from the measurement conveyor 2. The measurement conveyor 2 is, for its part, typically a belt conveyor.

The system of Figure 1 also comprises a measurement station 4, which in turn comprises cameras 5 and mirrors 6, the positioning of which defines the measurement station's 4 measurement area 7, within which measurement area 7 the pieces of sawn timber 3 can be imaged.

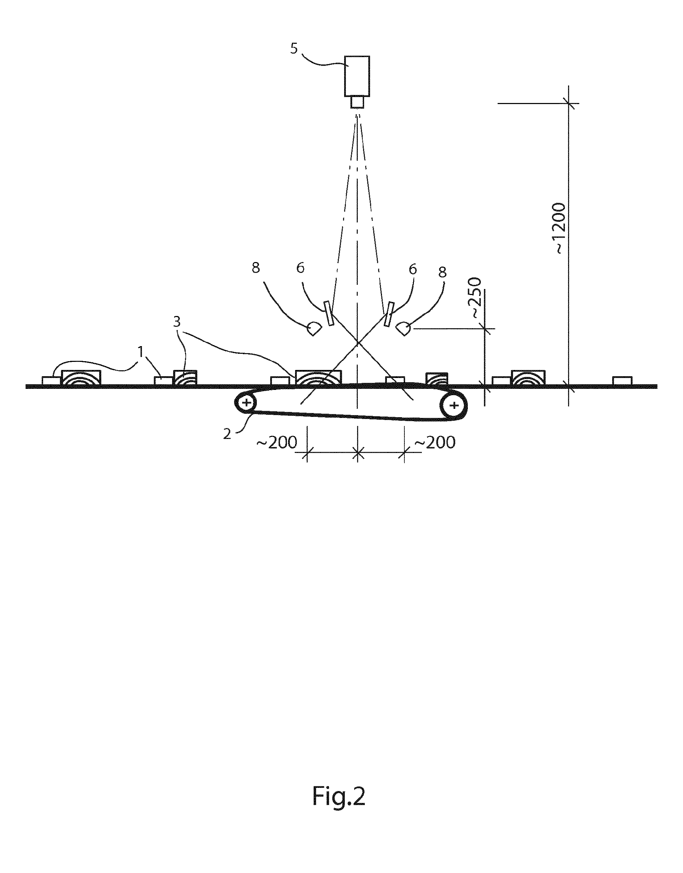

In Figure 2, the system of Figure 1 is viewed from the side, i.e. from the direction of the end of a piece of sawn timber 3 in the measuring area 7. The figure shows from the side direction the elements shown in Figure 1. In addition, the figure shows the lights 8, for illuminating the sawn timber 3, which belong to the measuring station 4. In the embodiment of Figure 2, the lights 8 illuminate the piece of sawn timber 3 from two different directions.

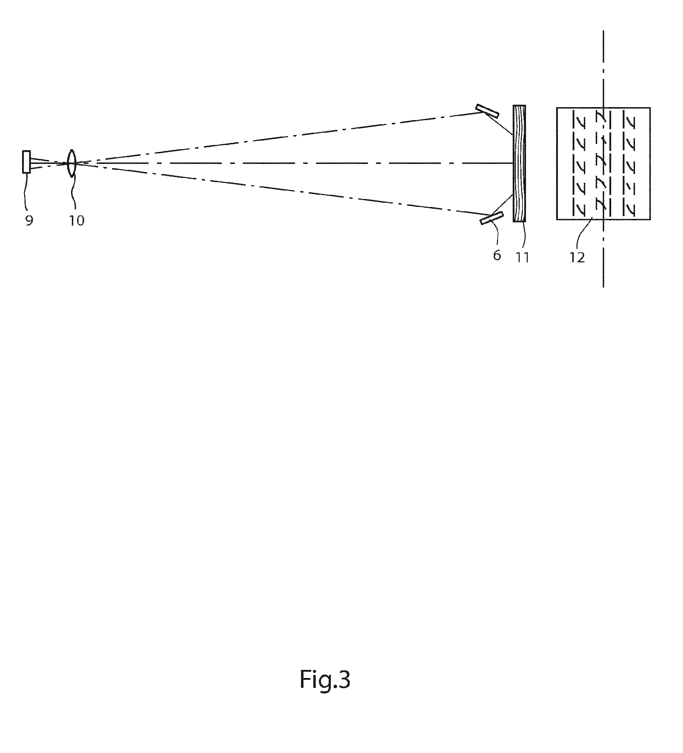

Figure 3 shows the creation of an image in the measuring station 4 of Figure 1. Figure 3 shows the matrix-camera element 9, the lens 10, and the surface 11 of the object being imaged (the top surface or bottom surface of the piece of sawn timber 3), as well as the image 12 of the object being imaged on a CCD or CMOS matrix of the matrix-camera element 9.

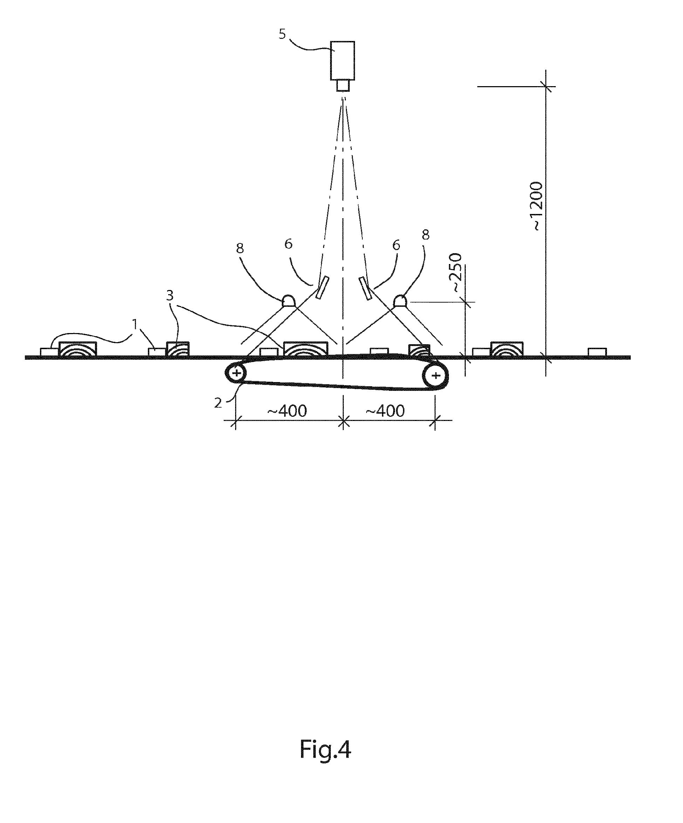

Figure 4 shows one possible variation of the arrangement of Figure 2. Common to both arrangements is that the cameras 5 image the piece of sawn timber 3 from directly between the mirrors 6 and, in addition, as two mirror images through the mirrors 6. The arrangements differ from each other in that, in the device of Figure 2, the mirror images are reflected from between the mirrors 6, and, in the device of Figure 4, from the opposite sides of the mirrors 6. In the device of Figure 2, the imaging areas formed by the direct imaging and the mirror images can partly overlap, the measurement area 7 being thus more compact. In the device of Figure 4 on the other hand, the direct imaging area and the mirror images are located at a distance from each other, so that the measurement area 7 is spread over a wider area.

Seen on the plane of the cross-sections of Figures 2 and 4, the cameras 5 image the piece of sawn timber 3 at essentially right angles. A possible variation is one in which the cameras 5 image the timber 3 being examined in the plane of the cross-sections of Figures 2 and 4 at other than essentially right angles, i.e. the cameras' imaging direction is deviated by tilting and moving the cameras towards the arrival or departure directions of the pieces of sawn timber.

One further possible variation to all of the embodiments described above is one in which only one mirror 6 or line of mirrors is used instead of two mirrors 6 or lines of mirrors.

Generally, sawn goods are imaged with the intention of automatically grading their quality and optimizing the pieces of sawn timber. By means of imaging, it is wished to determine, for example, each piece's width, thickness, length, and the width and depth of any waney edges. In addition, knots, resin pockets, splits, rot, bluing, and other factors affecting the quality and value of a sawn piece can be imaged from the faces and edges.

In the embodiments of the figures, the pieces of sawn timber 3 are transported on a main conveyor 1, i.e. a transverse scraper conveyor, which pushes the pieces forwards with the aid of chains and slats attached to them. The belts of the measurement conveyor 2 pull the pieces 3 off the slats of the main conveyor 1, so that their edges can be imaged unobstructedly. Nowadays, a typical speed is 100 - 200 pieces per minute. The traditional way is to image the pieces at two measuring stations and rotate the pieces of sawn timber mechanically between the stations. Using a three-direction measuring station, both sides and the end face of a piece can be imaged.

In the arrangement according to the embodiments, the pieces of sawn timber are imaged in three directions in a measuring station 4, using a single matrix camera, in such a way that the measuring areas 7 cover directly the upper surface of the piece and, with the aid of mirrors 6, both of its sides. The imaging matrices 9 of the camera can be, for example, CCD or CMOS or corresponding types.

With the aid of the embodiments described above a system can be implemented for the imaging of pieces of sawn timber 3 travelling transversely past the system, in which each piece 3 comprises a top surface and a bottom surface, as well as sides that connect them, the system being arranged to image the top surface and at least one side as the piece 3 travels past the measuring area 7 of the system.

In one embodiment, the system comprises at least one matrix camera 5, which is positioned in a first position relative to the path of the pieces of sawn timber 3, for imaging a piece of sawn timber 3. In addition, the system comprises at least one mirror 6, which is positioned in a second position relative to the path of the pieces 3 and the said first position. According to the embodiment, the first and second positions are selected in such a way that each piece of sawn timber 3 passing transversely can be imaged using the said at least one matrix camera 5 directly from a first direction and through the said at least one mirror 6 as a mirror image from at least one second direction, which is a different direction to the first direction.

According to one embodiment, the said at least one matrix camera 5 comprises two, three, or more matrix cameras 5, which are located in a transverse row. In this case, the transverse direction refers to the direction transverse to the direction of travel of the pieces of sawn timber, i.e. the longitudinal direction of the row is essentially parallel to the longitudinal direction of the pieces 3 being imaged.

According to one embodiment, the said at least one mirror 6 comprises two, three, or more mirrors, which are located in a transverse-direction row. In this case, the transverse direction refers to a direction corresponding to that described in the previous paragraph.

According to an embodiment, the mirrors 6 are placed out of the path of travel of the pieces of sawn timber, as is shown in Fig. 2. For example, the mirrors 6 are located above a level of the main conveyor 1 and also above the measurement conveyor 2 at a first height that is more than a designed greatest thickness of the pieces of sawn timber 3. At the same time, the height is also greater than the height of the slats 1. The first height is measured up to the lowest edge or part of the mirrors 6. The purpose of this design is to allow the pieces of sawn timber 3 to transversely pass the measuring station 4 without any obstruction by the mirrors 6.

According to an embodiment, the mirrors 6 are conventional mirrors including a reflecting metal surface and the path of light from the pieces of sawn timber 3 to the at least one matrix camera 5 comprises substantially one single reflection.

According to one embodiment, the system comprises at least one second mirror 6, which is positioned in a third position relative to the path of the pieces of sawn timber 3 and to the said first position. The third position is selected in such a way that each piece of sawn wood 3 travelling transversely past can be imaged using at least one matrix camera 5 through the said at least one second mirror 6, as a second mirror image from at least one third direction, which is a different direction to the first direction and the second directions.

According to one embodiment, the said at least one second mirror 6 comprises two, three, or more second mirrors 6, which are located in a transverse row in a corresponding manner to the first mirrors, but situated in a different location.

According to one embodiment, the first direction is substantially perpendicular to the direction of travel of the pieces of sawn timber and to at least a portion of the top surface of the piece 3, when the piece is in the measuring area of a respective at least one matrix camera 5. Positioning of this kind is shown in Figures 2 and 4.

According to a second embodiment, the first direction is tilted towards the direction of approach or departure of the piece of sawn timber 3 from the direction which is substantially perpendicular to the direction of travel of the piece and at least a portion of the top surface of the piece 3, when the piece is in the measuring area 7 of the corresponding at least one matrix camera 5.

According to one embodiment, the second direction is tilted at an angle, relative to the first direction, facing the direction of approach or departure of the sawn piece 3.

According to one embodiment, the said at least one matrix camera 5 comprises two, three, or more matrix cameras 5, which are situated in a first transverse row, and correspondingly the said at least one mirror 6 also comprises two, three, or more mirrors 6, which are situated in a second transverse row. In addition, the system comprises second two, three, or more mirrors 6, which are situated in a third transverse row, and the first, second, and third rows are positioned in such a way that the measuring area 7 is situated substantially between the second and third rows, seen from the direction of the first row. One such embodiment is shown in Figure 2.

According to one embodiment, the system comprises lights 8, which are arranged to illuminate a sawn piece 3 in the measuring area 7.

According to one embodiment, the lights 8 are positioned in a single row of lights, which illuminates the sawn piece 3 from one direction. Such an embodiment is suitable for use, for example, when the measuring station 4 comprises a single row of mirrors. Of course, in that case the entire system preferably comprises two measuring stations 4, between which the sawn piece 3 is rotated. I.e., in the system's two measuring stations, the upper face and one side are measured in both stations (the sawn piece being rotated between the measuring stations). One row of lights of each measuring station 4 is situated directly between the view of the matrix camera 5 and the view coming through the mirror 6, or close to the direction of the axis of symmetry of these views.

According to a second embodiment, the lights 8 are positioned in two rows of lights, which are arranged to illuminate a sawn piece 3 in the measuring area 7 from two different directions. Figure 2 and Figure 4 show such an embodiment.

According to a third embodiment, the lights 8 are situated in more than two rows of light, which are arranged to illuminate a sawn piece 3 in the measuring area 7 from more than two different directions.

According to one embodiment, the system comprises a second corresponding group of matrix cameras 5 and mirrors 6 positioned on the path of the sawn pieces 3 between the first group and the sawn-piece rotating device, in such a way that, with the aid of the first group, the top surface and at least one side of each sawn piece 3 can be imaged and, with the aid of the second group, the bottom surface and at least the second side of each sawn piece 3 can be imaged.

According to an embodiment, the system is adapted to image the pieces of sawn timber 3 during their transverse movement past the measuring area 7 of the system. This means that the operation is continuous. Thus, the main conveyor 1 and/or the measurement conveyor 2 are not stopped for the imaging operation and the pieces of sawn timber are under movement when they are imaged.

According to an embodiment, there is provided a system for imaging pieces of sawn timber that travel past the system transversely, wherein each piece of sawn timber comprises a top surface and a bottom surface as well as sides connecting the top and bottom surfaces. The system is adapted to image the top surface and at least one side as the piece of sawn timber travels past a measuring area of the system. The system comprises at least one matrix camera for imaging the pieces of sawn timber. The at least one matrix camera positioned in a first position relative to a path of the travel of the pieces of sawn timber. The system also comprises at least one mirror positioned in a second position relative to the first position and the path of the travel of the pieces of sawn timber. The second position is above the top surfaces of the pieces of sawn timber travelling past the system. According to this embodiment, the first and second positions are arranged to allow imaging of each piece of sawn timber from a first direction and a second direction. The first direction is from the top surface of the piece of sawn timber directly to the at least one matrix camera and the second direction is from one of the sides of the piece of sawn timber to the at least one mirror and further as a reflected mirror image to the at least one matrix camera.

According to another embodiment, there is provided a system for imaging pieces of sawn timber that travel past the system transversely, wherein each piece of sawn timber comprises a top surface and a bottom surface as well as sides connecting the top and bottom surfaces. The system is adapted to image the top surface and at least one side as the piece of sawn timber travels past a measuring area of the system. According to this embodiment, the system comprises at least one matrix camera for imaging the pieces of sawn timber, which at least one matrix camera is positioned in a first position relative to a path of the travel of the pieces of sawn timber. The system further comprises at least one mirror positioned in a second position relative to the first position and the path of the travel of the pieces of sawn timber, the second position being above the top surfaces of the pieces of sawn timber travelling past the system. In the embodiment, the first and second positions are arranged to allow imaging of each piece of sawn timber from a first direction and a second direction such that the first direction is from the top surface of the piece of sawn timber directly to the at least one matrix camera. On the other hand, the second direction is from one of the sides of the piece of sawn timber to the at least one mirror and further as a reflected mirror image to the at least one matrix camera such that the reflected mirror image is formed by a single reflection.

On the basis of the examples described above, it is obvious that, within the scope of the invention, it is possible to implement numerous solutions differing from the embodiments described above. Thus, it is not intended to restrict the invention to relate to only the aforementioned examples; instead the patent protection should be examined to the full extent of the accompanying Claims.

## Claims

1. A system for imaging pieces of sawn timber (3) that travel past the system transversely, wherein each piece of sawn timber (3) comprises a top surface and a bottom surface as well as sides connecting them, and which system is arranged to image the top surface and at least one side as the piece of sawn timber (3) travels past a measuring area (7) of the system, **characterized in that** the system comprises:
- at least one matrix camera (5) for imaging the piece of sawn timber (3), the at least one matrix camera (5) positioned in a first position relative to a path of the travel of the pieces of sawn timber (3), and
- at least one mirror (6) positioned in a second position relative to the path of the pieces of sawn timber (3) and to the said first position,
said first and second positions selected such that each piece of sawn timber (3) passing transversely can be imaged using the said at least one matrix camera (5) directly from a first direction and through the said at least one mirror (6) as a mirror image from at least one second direction.

2. The system according to Claim 1, **characterized in that** the said at least one matrix camera (5) comprises two, three, or more matrix cameras (5), which are situated in a transverse row.

3. The system according to Claim 1 or 2, **characterized in that** the said at least one mirror (6) comprises two, three, or more mirrors (6), which are situated in a transverse row.

4. The system according to any one of Claims 1 - 3, **characterized in that** the system comprises at least one second mirror (6), which is positioned in a third position relative to the path of the pieces of sawn timber (3) and to the said first position, which third position is selected such that each piece of sawn timber (3) passing transversely can be imaged using the said at least one matrix camera(5) as a second mirror image through the said at least one second mirror (6) from at least one third direction.

5. The system according to Claim 4, **characterized in that** the said at least one second mirror (6) comprises two, three, or more second mirrors (6), which are situated in a transverse row.

6. The system according to any one of Claims 1 - 5, **characterized in that** the first direction is substantially at right angles to the direction of travel of a piece of sawn timber and to at least a portion of the top surface of the piece of sawn timber (3) when the piece of sawn timber is in the measuring area of the corresponding at least one matrix camera (5).

7. The system according to any one of Claims 1 - 5, **characterized in that** the first direction is tilted towards the direction of arrival or departure of the piece of sawn timber (3) from a direction that is substantially perpendicular relative to the direction of travel of the piece of sawn timber and at least a portion of the top surface of the piece of sawn timber (3), when the piece of sawn timber is in the measuring area (7) of the corresponding at least one matrix camera (5).

8. The system according to any one of Claims 1 - 7, **characterized in that** the second direction is at a tilted angle directed towards the direction of arrival or departure of the piece of sawn timber (3), relative to the first direction.

9. The system according to any one of Claims 1 - 8, **characterized in that**
the said at least one matrix camera (5) comprises two, three, or more matrix cameras (5), which are situated in a first transverse row,
the said at least one mirror (6) comprises two, three, or more mirrors (6), which are situated in a second transverse row,
the said at least one mirror (6) comprises second two, three, or more mirrors (6), which are situated in a third transverse row, and
the first, second, and third rows are positioned such way that the measuring area (7) is situated substantially between the second and third rows, when seen from the direction of the first row.

10. The system according to any one of Claims 1 - 9, **characterized in that** it comprises lights (8), which are arranged to illuminate a piece of sawn timber (3) in the measuring area.

11. The system according to Claim 10, **characterized in that** the lights (8) are positioned in at least two transverse rows of light, in such a way that they illuminate a piece of sawn timber (3) in the measuring area from at least two different directions.

12. The system according to any one of Claims 1 - 10, **characterized in that** it comprises a second corresponding group of matrix cameras (5) and mirrors (6) positioned in the path of the pieces of sawn timber (3) after the first group and the sawn-piece rotation device, in such a way that, with the aid of the first group the top surface and at least one side of each piece of sawn timber (3) can be imaged and, with the aid of the second group the bottom surface and at least the second side of the piece of sawn timber (3) can be imaged.

13. The system according to any one of Claims 1 - 12, **characterized in that** the mirrors (6) are located at a first height above a level of the main conveyor (1) and a level of a measurement conveyor (2) for letting the pieces of sawn timber (3) supported by the main conveyor (1) and/or the measurement conveyor (2) pass transversely under the mirrors (6).

14. The system according to any one of Claims 1 - 13, **characterized in that** a path of light from the imaged pieces of sawn timber (3) to the at least one matrix camera (5) comprises one single reflection.

15. The system according to any one of Claims 1 - 14, **characterized in that** the system is adapted to image the pieces of sawn timber (3) during their transverse movement past the measuring area (7) of the system.
